# EUROPEAN PATENT APPLICATION

(11) **EP 1 250 882 A1**
(43) Date of publication of application: **23.10.2002**
(21) Application number: 01303666.0
(22) Date of filing: 20.04.2001
(51) Int. Cl.: A61B 1/07

(54) **Improvements relating to Endoscopes**

(71) Applicant: STMicroelectronics Limited, Edinburgh EH12 7BF (GB)
(72) Inventor: Raynor, Jeff, Edingburgh, EH12 5BD (GB)
(74) Representative: Murgitroyd, Ian G.

(57) **Abstract**

An endoscopic device(30,34) with a tubular casing (22) which defines a conduit for receiving an imaging device. The casing is constructed of a light-transmissive material such as a transparent rigid plastic. This allows illumination coupled into the casing (22) at one or more locations away from its distal end to be transmitted through the casing (20) and to exit its distal end. This provides illumination for an imaging device when positioned at the distal end of the casing (20) and removes the need to provide a separate light source un the housing of an endoscopic device (3).

## Description

This invention relates to endoscopes for use in imaging internal body organs.

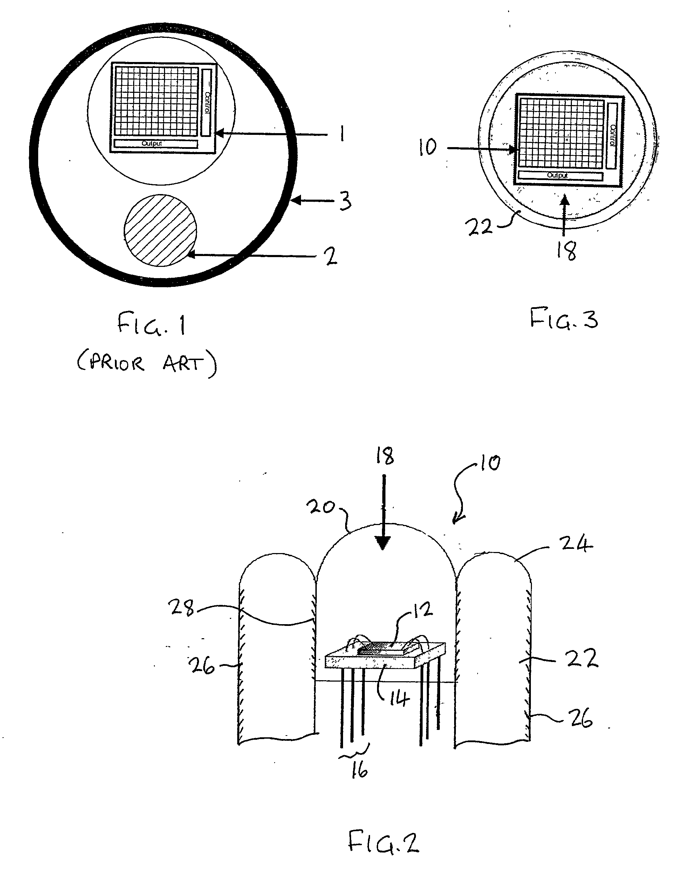

The use of endoscopes in investigatory and surgical procedures is well known. It is desirable for the cross-sectional size of the endoscope to be minimised. It is also necessary to provide illumination at the distal end of the endoscope to allow the image to be viewed, whether optically or by use of an image sensor located at the distal end. It is conventional to provide illumination from an external light source via a fibre optic cable within the endoscope. The necessity of positioning a fibre optic and, typically, a solid state image sensor chip at the distal end causes the overall diameter to be significantly larger than the size of the image sensor. This is illustrated in Figure 1, which shows an image sensor chip 1 and a fibre optic 2 disposed within a cylindrical steel housing 3.

Another area of existing technology which is relevant to the present invention is the question of providing sterile endoscopes. Most endoscopes using electronic image sensors currently in use are expensive items and it is therefore desired to use them repeatedly. However, the requirement for hospital sterilisation in itself increases the cost of the endoscope, as it must be designed for repeated sterilisation by heat or aggressive chemicals. There is therefore a need for designs which are suitable for producing endoscopes cheaply enough for the instruments to be disposable.

According to the present invention, an endoscopic device comprises a tubular casing defining a conduit for receiving an imaging device; the casing being of a light-transmissive material such that illumination coupled into said casing at one or more locations away from its distal end is transmitted through the casing and exits its distal end to provide illumination for an imaging device when positioned at said distal end.

The device may be implemented as a trocar or as an endoscope.

Other preferred features of the invention are defined in the claims.

An embodiment of the invention will now be described, by way of example only, with reference to the drawings, in which:
Figure 1 illustrates prior art designs, as discussed above;
Figure 2 is a schematic side view of part of an instrument embodying the invention;
Figure 3 is an end view corresponding to Figure 2, on a reduced scale;
Figure 4 is a schematic side view of the invention applied to a rigid endoscope;
Figure 5 is a similar view of the invention applied to a flexible endoscope; and
Figure 6 illustrates the invention applied to a trocar.

Referring to Figure 2, a solid state imaging system 10 of known type comprises a single chip image sensor 12 secured on a mounting board 14 and with leads 16 extending rearwardly. The imaging system 10 is encased in a clear plastic material 18, the front face of which is curved to form a spherical lens 20.

The assembly 10, 18 is mounted within a cylindrical casing 22 which provides the exterior of the distal end of an endoscope. The casing 22 is of a transparent plastic material and (not seen in Figure 1) is illuminated by a light source located away from the distal end. The light is transmitted along the casing 22 and emitted from the distal end face of the casing 22, which end face in this embodiment is radiused to form a projection lens 24 in the form of a half toroid.

Thus, the casing 22 acts as both the mechanical housing of the end of the endoscope, and as a light transmission path. The overall diameter of the instrument is therefore reduced, as seen in Figure 3, to a circle sufficient to encompass the imaging system 10 plus the thickness of the casing 22 which will typically be 1 - 2 mm.

As an alternative to the semi-toroidal lens shown, the end face of the casing 22 could be left plane, which would be less efficient in spreading the illumination, or could be formed with diffractive optics or the like, such as a grooved or stripe pattern, for example by etching, stamping, pressing or embossing, to spread the illumination; this could for example be a "Kinoform" pattern.

It may be possible to contain substantially all of the light within the casing 22 by internal reflection, but typically a reflective coating 26 will be applied to the external surface to prevent light loss, and a reflective coating 28 will be applied to the interface between the casing 22 and the encapsulation material 18 to prevent scattering of the supplied light onto the imaging system 12.

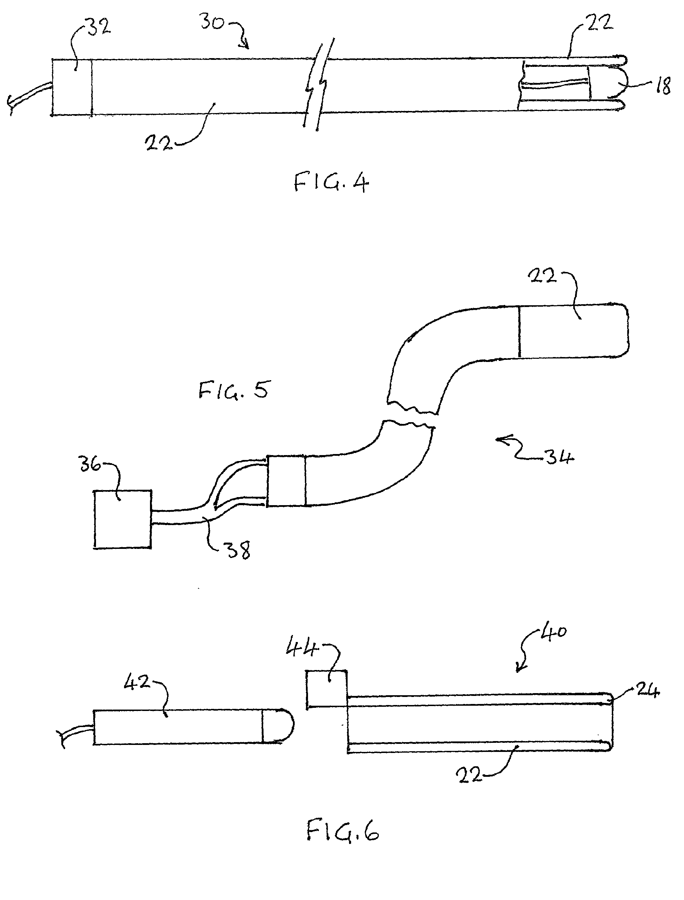

The invention may be applied to a solid endoscope 30 as seen in Figure 4, in which case the casing will extend along the whole length of the endoscope 30, and a light source 32 may be attached rigidly to the proximal end.

Alternatively, the invention may be applied to the distal end of a flexible endoscope 34 as seen in Figure 5, with the casing 22 forming a relatively short rigid section at the distal end. In this case, the light source could be situated immediately behind the casing 22 and powered by a flexible cable included in the flexible portion of the endoscope 34 or preferably, as shown, an external light source 36 may supply light to the casing 22 via a flexible light guide 38.

Light may be coupled into the transparent casing 22 from one or more light sources, which may be contained within the casing, at any location(s) away from the distal end of the casing 22.

Figure 6 illustrates a further embodiment, in which the invention is used in a trocar 40 which is used as an entry channel for one or more endoscopic instruments such as a camera 42. This is similar to Figure 1, in that the trocar 40 comprises a tubular casing 22 of rigid transparent plastic material formed with lens 24 and provided with reflective coatings 26 and 28. However, rather than having an imaging system permanently secured therein, the trocar 40 is positioned to give access to a body cavity, for example through the wall of the abdomen, and selected instruments can then access the body cavity through the trocar 40. An external light source 44 provides light through the casing 22 to illuminate the body cavity when a camera is inserted through the trocar 40.

The invention thus provides novel endoscopic instruments which permit reduction in instrument cross-section and more economic production.

## Claims

1. An endoscopic device comprising a tubular casing defining a conduit for receiving an imaging device; the casing being of a light-transmissive material such that illumination coupled into said casing at one or more locations away from its distal end is transmitted through the casing and exits its distal end to provide illumination for an imaging device when positioned at said distal end.

2. An endoscopic device according to claim 1, in which the casing is formed from a transparent rigid plastic material.

3. An endoscopic device according to claim 2, in which the distal end of the casing is formed to spread the exiting light.

4. An endoscopic device according to claim 3, in which said distal end is formed as a part-toroidal lens.

5. An endoscopic device according to claim 3, in which said distal end is formed with diffractive optics.

6. An endoscopic device according to any preceding claim, in which a reflective coating is provided on the exterior surface of the casing.

7. An endoscopic device according to any preceding claim, in which the internal surface of the casing adjacent the distal end thereof is provided with a reflective coating.

8. An endoscopic device according to any preceding claim, in the form of a trocar through which an imaging device and other instruments can be selectively inserted into and removed from a body cavity.

9. An endoscopic device forming an endoscope and comprising a device according to any of claims 1 to 7 having an imaging device secured therein adjacent said distal end.

10. An endoscope according to claim 9, in which the endoscope is rigid and the casing extends the whole length thereof.

11. An endoscope according to claim 9, in which the endoscope is flexible and said casing forms a distal end portion thereof.
